# EUROPEAN PATENT SPECIFICATION

(11) **EP 0 548 152 B1**
(45) Date of publication and mention of the grant of the patent: **24.07.1996**
(21) Application number: 91916072.1
(22) Date of filing: 12.09.1991
(51) Int. Cl.: A61M 15/00

(54) **AN INHALING DEVICE**
INHALIERVORRICHTUNG
DISPOSITIF D'INHALATION

(30) Priority: 12.09.1990 DK 2182/90; 20.02.1991 DK 297/91
(43) Date of publication of application: 30.06.1993
(73) Proprietor: BISGAARD, Hans, DK-2840 Holte (DK)
(72) Inventor: BISGAARD, Hans, DK-2840 Holte (DK)
(74) Representative: Plougmann, Vingtoft & Partners A/S
(86) International application number: PCT/DK91/00265
(87) International publication number: WO 92/04066

(56) References cited:
- EP-A- 0 384 050
- WO-A-90/07351
- US-A- 3 658 059
- US-A- 3 838 686
- US-A- 4 368 850
- US-A- 4 524 769
- US-A- 4 907 583

## Description

The present invention relates to an inhaling device for use in inhaling an active powdered or liquid substance from a reservoir, said device comprising a chamber into which the active substance may be sucked in a dispersed condition, and a mouthpiece by means of which a patient may inhale dispersed active substance from the dispersing chamber.

Various embodiments of inhaling devices or apparatuses of the above type are known, for example from US-4.907,583. The function of these known devices is conditioned by the creation of an airflow through the inhaling device caused by vigorous inhalation by the patient. This airflow causes active substance to be moved from the reservoir into the airflow in which it is dispersed. Thus, the efficiency of these known devices is to a high degree dependent on the patient's ability to produce a vigorous flow of inhalation air. This means that not only small children, but also adult patients having a lung function, which has been weakened for some reason or another, are unable to use these known inhalers or inhaling devices in an efficient manner.

Furthermore, WO-90/07351 discloses an inhaler which is operated by forcing or blowing air therethrough. Thus, a powdered medical product is supplied to and dispersed in air flowing from a pressurized air source such as an air pump, into a dispersing chamber via a Venturi throat. The dispersing chamber opens into the ambient atmosphere through a mouthpiece. In order to avoid waste of medical product a patient must at the same time inhale through the mouthpiece and operate the inhaler so as to generate the air flow through the inhaler.

Inhalers, the operation of which is based on the use of a gaseous propellant or a pressurized gas, such as freon, are also known. However, the use of such gaseous propellants often causes unwanted side effects for the patients.

The present invention provides an inhaling device of the above type by means of which a powdered or liquid active substance may be dispersed efficiently in the air or gas in the dispersing chamber of the device without using special gaseous propellants, even when the patient's lung function is weak or weakened.

The inhaling device according to the invention is characterized in that the dispersing chamber is defined by at least one wall part, which is movable between positions, in which the chamber attains a minimum volume and a maximum volume, respectively, and in moving means for biassing the movable wall part towards the position in which the dispersing chamber attains its maximum volume, so as to suck active substance into the dispersing chamber from the reservoir.

The movable wall part or parts may be moved towards the maximum volume by the moving means with a force or at a rate sufficient to create a suitable vacuum within the dispersing chamber. This causes air to flow from the ambient atmosphere into the dispersing chamber, and the airflow is sufficiently forceful to cause an efficient dispersion of the active substance supplied from the reservoir.

When the active substance has been dispersed in the air within the dispersing chamber the patient may without any difficulties inhale air and active substance from the dispersing chamber through the mouthpiece, whereby the active substance is inhaled into the patient's lungs.

The reservoir for active substance and the necessary means for moving this substance into the airflow being sucked into the dispersing chamber when moving the movable wall part or parts towards the maximum volume may be inherent parts of the inhaling device according to the invention. Alternatively, the dispersing chamber of the inhaling device according to the invention may be adapted to be connected to another separate inhaler of the type, the operation of which is based on the patient's inhalation ability, for example as described in Swedish patent specification No. 453,566.

When the patient inhales air and active substance from the dispersing chamber into the lungs, it should be possible to replace the inhaled air by air flowing into the dispersing chamber from the ambient atmosphere. Such a flow of replacement air may, for example, flow into the dispersing chamber through the said separate inhaler. The inhaling device may alternatively or additionally be provided with valve means for communicating the dispersing chamber with the ambient atmosphere immediately before or when said chamber attains its maximum volume. Such valve means may be operated manually or they may be adapted to open automatically when or immediately before the dispersing chamber attains its maximum volume.

The dispersing chamber may be formed by or comprise a compressible bulb, a collapsible bellows, or a similar pumping device comprising deformable wall parts which from a compressed or collapsed condition may be returned to a distended position under the influence of inherent resilent forces or by means of special elastic returning means. In the preferred embodiment, however, the dispersing chamber comprises a cylinder having a piston arranged therein so as to be displaceable between advanced and retracted positions.

The piston may be connected to a piston rod by means of which the piston may be moved manually between its advanced and retracted positions so that air including dispersed active substance is drawn into the dispersing chamber. In order to obtain a more uniform suction of air into the dispersing chamber and a more uniform dispersion of active substance in such air from time to time it is convenient, however, to move the piston from its retracted to its advanced position under the influence of a force, which may be kept substantially uniform from time to time and which may be chosen so as to obtain an airflow velocity which is optimum. Thus, the piston may be spring biassed towards its advanced position, releasable locking means being adapted to releasably retain the piston in its retracted position. The piston may then manually and against the spring bias be moved to its retracted position, in which the dispersing chamber attains its minimum volume, and the piston may be releasably locked in this position. When the locking means are released the piston is rather quickly returned to its advanced position under the influence of the spring bias, in which position the dispersing chamber attains its maximum volume. Such piston movement creates a vigorous flow of air towards the dispersing chamber into which airflow active substance is sucked from the reservoir.

At least one through-opening interconnecting the dispersing chamber with the ambient atmosphere may be defined in the wall of the cylinder at a location immediately inside the piston in the advanced position of the piston. In combination with the piston such openings may function as the valve means described above, because the dispersing chamber is automatically communicated with the atmosphere through such openings immediately prior to the time at which the dispersing chamber attains its maximum volume. When the said openings are passed or uncovered by the piston air may flow into the cylinder or dispersing chamber through said openings during the very last part of the piston movement because of the vacuum created in the dispersing chamber. Such air inflow creates turbulence within the dispersing chamber promoting the dispersion of the active substance in the air contained in the dispersing chamber.

As mentioned above, the patient may now inhale dispersed active substance from the dispersing chamber by means of a mouthpiece which may be permanently connected to the dispersing chamber, or which may be mounted thereon when the active substance has been sucked into the dispersing chamber. In order to prevent that the active substance is blown out from the dispersing chamber and wasted if the patient exhales or blows through the mouthpiece, the mouthpiece may communicate with the dispersing chamber through a one-way valve allowing flow of air out from the dispersing chamber only. The mouthpiece preferably communicates with an expiration passage including a one-way valve allowing air to flow out from the mouthpiece only. The patient may then freely inhale and exhale through the mouthpiece, and air being inhaled then comes from the dispersing chamber, while air being exhaled will flow out through the one-way valve in the expiration passage.

The reservoir or magazine from which the active substance is released may be adapted to release the active substance in metered standard doses suitable for use by an adult, vide for example the above mentioned Swedish patent. However, such standard dose is too big when the patient is a child. Therefore, according to the invention the dispersing chamber may comprise an inlet chamber adapted to communicate with the reservoir and a main chamber. The inlet chamber may then be removably connected to the main chamber via a connecting passage, and the mouthpiece may be adapted to be connected to the inlet chamber through the connecting passage, when the inlet chamber is separated from the main chamber. Alternatively, the inlet chamber may be communicating with the main chamber via a one-way valve allowing airflow from the inlet chamber to the main chamber, only. In this case the mouthpiece may be permanently or removably connected to the inlet chamber. When active substance has been dispersed in the air contained in the dispersing chamber comprising an inlet chamber and a main chamber, the inlet chamber may be removed from the main chamber, if desired, and provided with the mouthpiece if the mouthpiece has not already been arranged on the inlet chamber. The patient may then inhale air and the active substance dispersed therein from the inlet chamber, but not from the main chamber. The volume of the inlet chamber may be chosen so that the amount of active substance, which is dispersed in the air contained in the inlet chamber, is adjusted to the patient in question, who may, for example, be a child.

The inlet chamber may possibly be designed so that its volume may be adjusted. As an example, the inlet chamber may comprise a cylinder with a piston, which is arranged displaceably therein, and which may be locked in a desired position. In this manner the volume of the inlet chamber may be adapted to the child's age. If, the patient is a child who needs an amount of active substance corresponding to half the dose suitable for an adult, the volume of the inlet chamber may be adjusted so that it is substantially equal to the volume of the main chamber.

Alternatively, an inlet chamber with a substantially constant, unchangeable volume may be used. In this case, the inhaling device may comprise two or more inlet chambers having different volumes and/or shapes. It is then possible to select an inlet chamber having a volume suitable for the patient in question.

As an alternative to the use of different inlet chambers or an inlet chamber with an adjustable volume, the stroke of the piston in the cylinder defining the main chamber between its advanced and retracted position may be adjustable. Thus, the piston stroke is increased when the amount of active substance in the inlet chamber is to be reduced.

The inlet chamber may have any suitable shape. As an example, the inlet chamber may be spherical, circularly cylindrical or have an inner wall defining any other surface of revolution. In such case, where the inlet chamber has a cylindrical wall part, the reservoir is preferably connected to the inlet chamber through a passage, which is directed substantially peripherally in relation to the cylindrical wall part so as to create a whirling airflow pattern therein promoting dispersion of the active substance.

As mentioned above the mouthpiece may be permanently connected to the inlet chamber, but the mouthpiece is preferably removably connected to the inlet chamber, so that the same mouthpiece may be used for different inlet chambers. When the inlet chamber has been separated from the main chamber the section of the connecting passage formed on the inlet chamber may be closed by a suitable lid, and the mouthpiece may then be mounted at another position of the inlet chamber. Alternatively, the said connecting passage section may contain a one-way valve preventing air from being sucked into the inlet chamber through this connecting passage section.

The mouthpiece may be integral with a unit comprising the expiration passage and the one-way valve arranged therein so that the patient may inhale the air and the dispersed active substance contained in the inlet chamber by inhaling and exhaling or naturally breathing through the mouthpiece.

The spring biassing the piston from its advanced to its retracted position may be so strong that it is difficult or impossible to manually move the piston against the spring bias without using special moving means. As an example, the inhaling device may further comprise a lever device for moving the piston against the spring bias from its advanced to its retracted position.

The invention will now be further described with reference to the drawings, wherein
Fig. 1 is a side view and partially sectional view of an embodiment of the inhaling device according to the invention,
Fig. 2 illustrates the same as fig. 1 immediately after operation of the inhaling device,
Fig. 3 is a perspective view of the inhaling device shown in Figs. 1 and 2, on which an inlet chamber forming part of the dispersing chamber has been mounted, and
Fig. 4 is a perspective view showing the inlet chamber provided with a mouthpiece removed from the main chamber.

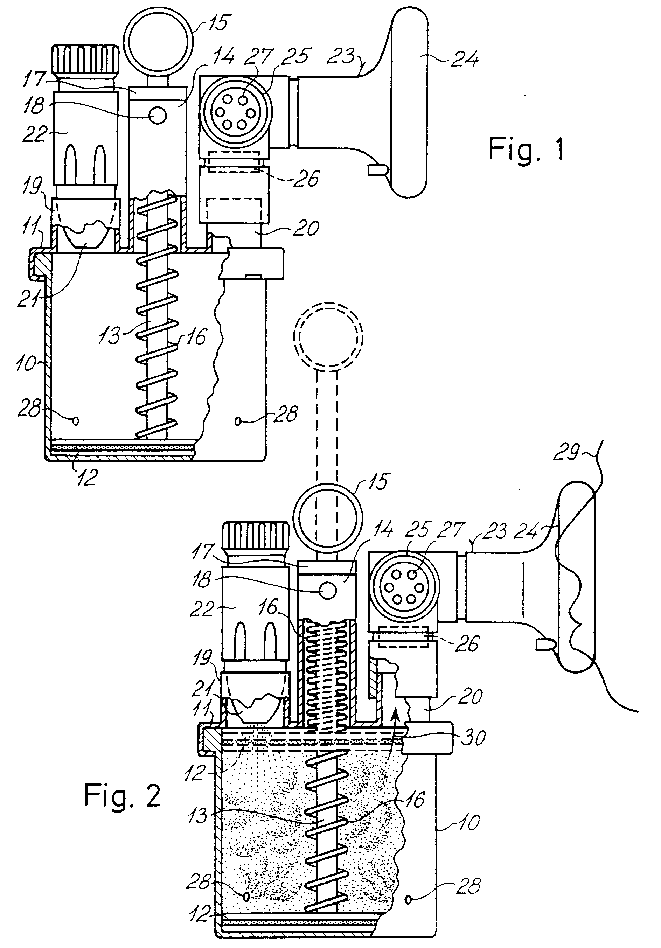

The inhaler shown in the drawings comprises a cylindrical container or a cylindrical dispersing chamber 10 closed at the top end by means of a lid 11. A piston 12 (Figs. 1 and 2) is displaceably arranged within the cylindrical container 10. The piston is connected to a piston rod 13 and moveable between an upper position indicated by dotted lines in Fig. 2, and a lower position shown in solid lines in Figs. 1 and 2. The piston rod 13 extending upwardly through a tubular part 14 formed on the lid 11 is provided with a gripping portion 15, which may be shaped as a ring as shown in the drawings. A coil spring 16 surrounding the piston rod 13 and having its opposite ends engaging with the upper surface of the piston 12 and an upper end wall 17 of the tubular portion 14, is biassing the piston 12 towards its advanced position or bottom position shown in Fig. 1. The piston 12 may be releasably locked in its upper position, which is shown in dotted lines in Fig. 2, by means of a releasable locking member 18. As shown, the locking member 18 may be positioned in the upper part of the tubular portion 14 and be adapted to cooperate with the piston rod 13. As an example, the locking member may be a retaining member which is arranged displaceably in relation to the tubular portion 14 and which may engage with a shoulder or abutment surface formed on the piston rod.

Furthermore, a pair of upwardly directed tube sections 19 and 20, respectively, are formed on the lid 11. As shown in Figs. 1 and 2 the tube section 19 is adapted to receive a mouthpiece 21 of a conventional inhaler 22, which may, for example, be of the type disclosed in Swedish patent No. 453,566. A mouthpiece unit 23 comprising a mouthpiece or a mask 24 is removeably mounted on the tube section 20. The mouthpiece unit 23 defines an inhalation passage connecting the mask 24 with the inner space of the cylindrical container 10 via the tube section 20. The mouthpiece unit 23 further comprises an expiration branch 25 defining an expiration passage, which is separated from the inhalation passage. The expiration passage contains a one-way valve 26, which may, for example, be a flap valve allowing air to flow through the inhalation passage from the container 10 towards the mask or mouthpiece 24, but prevents airflow in the opposite direction. The expiration passage also contains a one-way valve 27 allowing air to flow out from the expiration passage, while air is prevented from flowing from the ambient atmosphere into and through the expiration passage.

For normal use of the inhaler 22 the mouthpiece 21 is inserted between a patient's lips. Thereafter, the patient inhales vigorously, whereby a standard dose of a powdered inhalation substance is dispersed in the air being caused to flow through the inhaler 22. However, some patients, such as children and adults having a weakened lung function, are not able to inhale sufficiently forcefully to secure that the inhaler 22 operates at its optimum. This problem may be solved by using the inhaling device shown in Figs. 1 and 2. The inhaling device may be operated as follows:

The piston 12 may manually be moved to its upper position indicated in Fig. 2 in dotted lines, by means of the gripping portion 15, whereby the coil spring 16 is axially compressed. The piston and the piston rod may be locked in this retracted position by means of the locking member 18. When the locking member 18 is released, the piston 12 is moved quickly towards its lower, advanced position shown in solid lines in Figs. 1 and 2, under the influence of the bias of the spring 16. Because the removable lid 11 is sealingly connected to an upper rim portion of the container 10, and because the one-way valve 26 prevents air from the ambient atmosphere from being sucked through the mouthpiece unit 23, the sudden downward movement of the piston 12 creates a vacuum within the cylindrical container 10. This vacuum creates a forceful flow of air through the inhaler 22. Consequently, the said standardized dose of inhaling substance is dispersed in the air sucked into the container 10. The cylindrical wall of the container 10 may be provided with one or more through openings 28 at its lower end. Preferably, all of these openings are positioned with substantially the same small spacing from the container bottom. When the piston 12 has passed these openings during its downward movement air may also flow into the container through these openings 28, whereby increased turbulence is created within the container 10, and such turbulence contributes to an improved dispersion of the powdered inhaling substance in the air contained in the container. The mask 24 may now be placed against a patient's 29 face (Fig. 2) so that it tightly engages the patient's face around the nose and mouth, and the patient may thereafter breath deeply one or several times, whereby air with active substance dispersed therein is sucked into the patient's lungs from the container 10. When the patient is inhaling, air flows from the container 10 through the tube length 20, the inhalation passage of the mouthpiece unit 23 and the one-way valve 26 arranged therein and down into the patient's lungs as indicated by an arrow 30 in Fig. 2. When the patient is exhaling air flows from the patient's lungs through the one-way valve 27 and to the ambient atmosphere through the expiration branch 25. The one-way valve 26 prevents, however, that expiration air flows into the container 10. Now, a new standard dose of inhaling substance may be advanced by means of a metering device included in the inhaler 22, and thereafter the inhaling device may again the operated as described above.

The standard dose of inhaling substance suitable for a grown-up patient substantially exceeds the suitable dose, when the patient is a child. The dose of inhaling substance could, of course, be adapted to the child's height or age. However, this would mean that it would be necessary to market a number of different inhalers 22 including different doses. This problem may be solved by means of the inhaling device according to the invention.

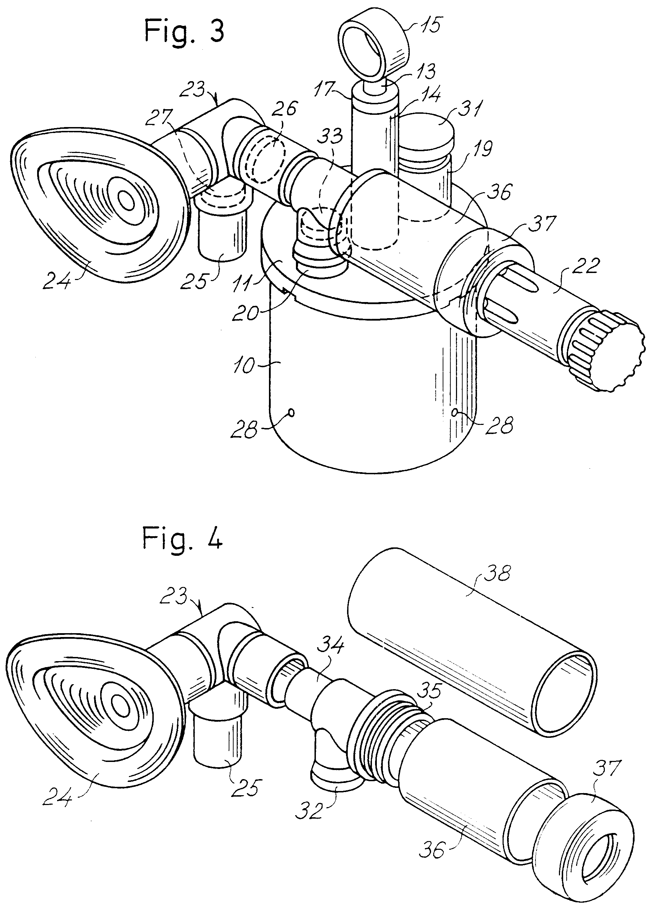

Fig. 3 shows the inhaling device according to Figs. 1 and 2, where the inhaler 22 has been removed from the tube section 19, which has been closed by means of a lid or stopper 31. The mouthpiece unit 23 has been removed from the tube section 20 and replaced by a T-piece 32 containing a one-way valve 33 allowing air to flow through the free legs or branches of the T-piece and down into the container 10, but not in the opposite direction. The mouth-piece unit 23 is mounted on one branch 34 of the T-piece, while an auxiliary cylinder or an inlet chamber 36 is mounted on the other branch 35 of the T-piece. The inner wall of the cylinder 36 is in sealing contact with the outer surface of the branch 35 which may be provided with peripheral sealing lips or beads. An annular sleeve 37 made from rubber, plastic or another resilient material may be mounted on the other end of the auxiliary cylinder 36 and defines an opening for sealingly receiving the mouthpiece 21 of the inhaler 22 therein.

The inhaling device shown in Fig. 3 may be operated in a manner similar to that described above. When the piston 12 is moved to its lower advanced position under the influence of the bias of the coil spring 16 the vacuum created within the container 10 causes air to flow through the inhaler 22, and a standardized dose of the inhaling substance is dispersed in the air flow. Thus, air with inhaling substance dispersed therein will fill not only the inlet chamber or auxiliary cylinder 36, but also the cylindrical container or main chamber 10. Now air and inhaling substance contained in the auxiliary cylinder or the inlet chamber 36 may be inhaled by a patient inhaling through the mask 24 in a manner described above. However, the patient cannot inhale air and inhaling substance contained in the main chamber. If desired, the T-piece 32 and the parts mounted thereon may be removed from the tube section 20 before the patient inhales air from the auxiliary cylinder 36. The relationship between the volume of the auxiliary cylinder or inlet chamber 36 and the volume of the main chamber 10 may be chosen so that the patient may inhale a desired fraction of the standardized dose of inhaling substance, which is released by the inhaler 22. The auxiliary cylinder or the inlet chamber 36 may be replaced by another auxiliary cylinder 38 having a volume which is larger or smaller than the volume of the auxiliary cylinder 36, whereby the amount of active substance inhaled by the patient from the auxiliary cylinder or inlet chamber is adapted to the child's age and/or height.

It should be understood that various changes of the embodiment shown in the drawings may be made within the scope of the present invention. As an example, the cylindrical container 10 may be replaced by a bellows or a compressible bulb by means of which a dose of inhaling substance may be sufficiently forcibly sucked from the inhaler 22. It should also be mentioned that the inhaler 22 may be of any type which is adapted to function when the patient is inhaling air therethrough. While the inhaling device according to the invention has especially been disclosed with reference to inhalers for use in connection with powdered inhaling substances, it should be understood that the inhaling device according to the invention may also be used in connection with inhalers operating with liquid inhaling means or substances.

## Claims

1. An inhaling device for use in inhaling an active powdered or liquid substance from a reservoir (22), said device comprising a chamber (10, 36, 38) into which the active substance may be sucked in a dispersed condition, and a mouthpiece (24) by means of which a patient (29) may inhale dispersed active substance from the dispersing chamber (10, 36, 38),
**characterized** in that the dispersing chamber (10, 36, 38) is defined by at least one wall part (12), which is movable between positions, in which the chamber (10, 36, 38) attains a minimum volume and a maximum volume, respectively, and in moving means (16) for biassing the movable wall part (12) towards the position in which the dispersing chamber attains its maximum volume, so as to suck active substance into the dispersing chamber from the reservoir (22).

2. An inhaler according to claim 1, wherein the dispersing chamber (10, 36, 38) is adapted to be connected to a conventional inhaler (22).

3. An inhaling device according to claim 1 or 2, further comprising valve means (12, 28) for communicating the dispersing chamber (10, 36, 38) with the ambient atmosphere immediately before or when said chamber attains its maximum volume.

4. An inhaling device according to any of the claims 1-3, wherein the dispersing chamber (10, 36, 38) comprises a cylinder (10) having a piston (12) arranged therein so as to be displaceable between advanced and retracted positions.

5. An inhaling device according to claim 4, wherein the piston (12) is spring biassed towards its advanced position, releasable locking means (18) being adapted to releasably retain the piston in its retracted position.

6. An inhaling device according to claim 3 and 4 or 5, wherein at least one through-opening (28) interconnecting the dispersing chamber (10, 36, 38) with the ambient atmosphere is defined in the wall of the cylinder (10) at a location immediately inside the piston (12) in the advanced position of the piston.

7. An inhaling device according to any of the claims 1-6, wherein the mouthpiece (24) communicates with the dispersing chamber (10, 36, 38) through a one-way valve (26) allowing flow of air out from the dispersing chamber (10, 36, 38) only.

8. An inhaling device according to claim 7, wherein the mouthpiece is communicating with an expiration passage (25) including a one-way valve (27) allowing air to flow out from the mouthpiece, only.

9. An inhaling device according to any of the claims 1-8, wherein the dispersing chamber (10, 36, 38) comprises an inlet chamber (36, 38) adapted to communicate with the reservoir, and a main chamber (10).

10. An inhaling device according to claim 9, wherein the inlet chamber (36, 38) is communicating with the main chamber (10) via a one-way valve (33) allowing airflow from the inlet chamber to the main chamber, only.

11. An inhaling device according to claim 9 or 10, wherein the inlet chamber (36, 38) is removably connected to the main chamber (10).

12. An inhaling device according to any of the claims 9-11, wherein the volume of the inlet chamber is adjustable.

13. An inhaling device according to any of the claims 9-11, wherein the volume of the inlet chamber (36, 38) is substantially constant.

14. An inhaling device according to claim 13, further comprising at least two interchangeable inlet chambers (36, 38) having different volumes and/or shapes.

15. An inhaling device according to claim 4 and any of the claims 9-14, wherein the stroke of the piston (12) in the cylinder (10) defining the main chamber between its advanced and retracted positions is adjustable.

16. An inhaling device according to any of the claims 9-15, wherein the inlet chamber has a cylindrical wall part, the reservoir being connected to the inlet chamber through a passage, which is directed substantially peripherally in relation to the cylindrical wall part.

17. An inhaling device according to any of the claims 8-16, wherein the mouthpiece is integral with a unit (23) comprising the expiration passage (25) and the one-way valve (27) arranged therein.

18. An inhaling device according to any of the claims 5.17, further comprising a lever device for moving the piston against the spring bias from its advanced to its advanced retracted.

## Patentansprüche

1. Inhaliervorrichtung zur Verwendung beim Inhalieren von aktiven pulverisierten oder flüssigen Substanzen aus einem Behälter (22), wobei die Vorrichtung eine Kammer (10, 36, 38), in welche die aktive Substanz in einem dispergierten Zustand eingesaugt werden kann, und ein Mundstück (24) umfaßt, mit dem ein Patient (29) dispergierte aktive Substanz von der Dispersionskammer (10, 36, 38) inhalieren kann, gekennzeichnet dadurch, daß die Dispersionskammer (10, 36, 38) mindestens durch einen Wandteil (12), der zwischen Positionen, in welchen die Kammer (10, 36, 38) ein minimales Volumen beziehungsweise ein maximales Volumen erreicht, definiert ist, und durch eine Bewegungsvorrichtung (16) zur Vorspannung des beweglichen Wandteils (12) in eine Position, in der die Dispersionskammer ihr maximales Volumen erreicht, um so aktive Substanz aus dem Behälter (22) in die Dispersionskammer zu saugen.

2. Inhaliervorrichtung nach Anspruch 1, wobei die Dispersionskammer (10, 36, 38) so ausgebildet ist, daß sie mit einem konventionellen Inhalierer (22) verbunden werden kann.

3. Inhaliervorrichtung nach Anspruch 1 oder 2, weiterhin enthaltend Ventilvorrichtungen (12, 28) zur Verbindung der Dispersionskammer (10, 36, 38) mit der Umgebungsatmosphäre, direkt bevor oder wenn die Kammer ihr maximales Volumen erreicht.

4. Inhaliervorrichtung nach einem der Ansprüche 1 - 3, wobei die Dispersionskammer (10, 36, 38) einen Zylinder (10) umfaßt, der einen darin angeordneten Kolben (12) aufweist, so daß dieser zwischen einer vorgerückten und rückgezogenen Position bewegbar ist.

5. Inhaliervorrichtung nach Anspruch 4, wobei der Kolben (12) mit einer Feder in seine vorgerückte Position gedrückt wird, und eine lösbare Verriegelungsvorrichtung (18) so ausgebildet ist, daß sie den Kolben in seiner rückgezogenen Position lösbar zurückhält.

6. Inhaliervorrichtung nach Anspruch 3 und 4 oder 5, wobei mindestens eine die Dispersionskammer (10, 36, 38) mit der Umgebungsatmosphäre verbindende Durchgangsöffnung (28) in der Wand des Zylinders (10) an einem Ort direkt innerhalb des Kolbens (12) in vorgerückter Position des Kolbens ausgebildet ist.

7. Inhaliervorrichtung nach einem der Ansprüche 1 - 6, wobei das Mundstück (24) mit der Dispersionskammer (10, 36, 38) durch ein Einwegeventil (26) verbunden ist, das den Luftfluß nur aus der Dispersionskammer (10, 36, 38) heraus gestattet.

8. Inhaliervorrichtung nach Anspruch 7, wobei das Mundstück mit einem Ausatemkanal (25) verbunden ist, der ein Einwegeventil (27) umfaßt, das den Luftfluß nur aus dem Mundstück heraus gestattet.

9. Inhaliervorrichtung nach einem der Ansprüche 1 - 8, wobei die Dipersionskammer (10, 36, 38) eine Einlaßkammer (36, 38), die so ausgebildet ist, daß sie mit dem Behälter in Verbindung steht, und eine Hauptkammer (10) umfaßt.

10. Inhaliervorrichtung nach Anspruch 9, wobei die Einlaßkammer (36, 38) mit der Hauptkammer (10) über ein Einwegeventil (33) verbunden ist, das den Luftfluß nur von der Einlaßkammer in die Hauptkammer gestattet.

11. Inhaliervorrichtung nach den Ansprüchen 9 oder 10, wobei die Einlaßkammer (36, 38) abnehmbar mit der Hauptkammer (10) verbunden ist.

12. Inhaliervorrichtung nach einem der Ansprüche 9 - 11, wobei das Volumen der Einlaßkammer einstellbar ist.

13. Inhaliervorrichtung nach einem der Ansprüche 9 - 11, wobei das Volumen der Einlaßkammer (36, 38) im wesentlichen konstant ist.

14. Inhaliervorrichtung nach Anspruch 13, weiterhin enthaltend mindestens zwei auswechselbare Einlaßkammern (36, 38), die verschiedene Volumen und/oder Formen aufweisen.

15. Inhaliervorrichtung nach Anspruch 4 und einem der Ansprüche 9 - 14, wobei der Hub des Kolbens (12) im Zylinder (10), der die Hauptkammer zwischen seiner vorgerückten und rückgezogenen Position festlegt, einstellbar ist.

16. Inhaliervorrichtung nach einem der Ansprüche 9 - 15, wobei die Einlaßkammer einen zylindrischen Wandteil aufweist, und der Behälter mit der Einlaßkammer durch einen Durchgang verbunden ist, der relativ zum zylindrischen Wandteil im wesentlichen peripher ausgerichtet ist.

17. Inhaliervorrichtung nach einem der Ansprüche 8 - 16, wobei das Mundstück integral mit einer Einheit (23) ausgebildet ist, die den Ausatemkanal (25) und das darin angeordnete Einwegeventil (27) umfaßt.

18. Inhaliervorrichtung nach einem der Ansprüche 5 - 17, weiterhin enthaltend eine Hebelvorrichtung zur Bewegung des Kolbens entgegen der Federkraft von seiner vorgerückten in seine rückgezogene Position.

## Revendications

1. Un dispositif d'inhalation destiné à être utilisé pour inhaler une substance active en poudre ou liquide depuis un réservoir (22), ledit dispositif comprend une chambre (10, 36, 38), dans laquelle la substance active peut être aspirée à l'état dispersé et d'un embout buccal (24) par l'intermédiaire duquel un patient (29) peut inhaler la substance active dispersée depuis la chambre de dispersion (10, 36, 38) caractérisé en ce que la chambre de dispersion (10, 36, 38) est définie par au moins une partie formant paroi (12) qui est déplaçable entre les positions dans lesquelles la chambre (10, 36, 38) atteint un volume minimum et un volume maximum, respectivement, et par des moyens de déplacement (16) pour solliciter la partie formant paroi mobile (12) vers la position dans laquelle la chambre de dispersion atteint son volume maximum, de façon à aspirer de la substance active dans la chambre de dispersion depuis le réservoir (22).

2. Un dispositif d'inhalation selon la revendication 1, dans lequel la chambre de dispersion (10, 36, 38) est conçue pour être raccordée à un inhalateur (22) classique.

3. Un dispositif d'inhalation selon la revendication 1 ou 2, comportant en outre des moyens de valve (12, 28) pour mettre en communication la chambre de dispersion (10, 36, 38) avec l'atmosphère ambiante immédiatement avant que ladite chambre n'atteigne ou au moment où elle atteint son volume maximum.

4. Un dispositif d'inhalation selon l'une quelconque des revendications 1-3, dans lequel la chambre de dispersion (10, 36, 38) se compose d'un cylindre (10) comportant un piston (12) disposé dans celui-ci de manière à pouvoir être déplacé entre des positions avancée et reculée.

5. Un dispositif d'inhalation selon la revendication 4, dans lequel le piston (12) est sollicité par un ressort dans sa position avancée, des moyens de verrouillage déblocables (18) étant adaptés pour maintenir, avec déblocage possible, le piston dans sa position reculée.

6. Un dispositif d'inhalation selon la revendication 3, 4 ou 5, dans lequel au moins une ouverture traversante (28) mettant en communication la chambre de dispersion (10, 36, 38) avec l'atmosphère ambiante est ménagée dans la paroi du cylindre (10) à un endroit se situant immédiatement à l'intérieur du piston (12) dans la position avancée du piston.

7. Un dispositif d'inhalation selon l'une quelconque des revendications 1 à 6, dans lequel l'embout buccal (24) communique avec la chambre de dispersion (10, 36, 38) en passant par un clapet de retenue (26) permettant au flux d'air de sortir de la chambre de dispersion (10, 36, 38) uniquement.

8. Un dispositif d'inhalation selon la revendication 7, dans lequel l'embout buccal communique avec un passage d'expiration (25) comportant un clapet de retenue (27) permettant au flux d'air de sortir de l'embout buccal uniquement.

9. Un dispositif d'inhalation selon l'une quelconque des revendications 1 à 8, dans lequel la chambre de dispersion (10, 36, 38) comprend une chambre d'entrée (36, 38) conçue pour communiquer avec le réservoir ainsi qu'une chambre principale (10).

10. Un dispositif d'inhalation selon la revendication 9, dans lequel la chambre d'entrée (36, 38) communique avec la chambre principale (10) par l'intermédiaire d'un clapet de retenue (33) permettant au flux d'air provenant de la chambre d'entrée de passer dans la chambre principale uniquement.

11. Un dispositif d'inhalation selon la revendication 9 ou 10, dans lequel la chambre d'entrée (36, 38) est raccordée de façon amovible à la chambre principale (10).

12. Un dispositif d'inhalation selon l'une quelconque des revendications 9 à 11, dans lequel le volume de la chambre d'entrée est réglable.

13. Un dispositif d'inhalation selon l'une quelconque des revendications 9-11, dans lequel le volume de la chambre d'entrée (36, 38) est sensiblement constant.

14. Un dispositif d'inhalation selon la revendication 13, comportant en outre au moins deux chambres d'entrée (36, 38) interchangeables ayant des volumes et/ou des formes différents.

15. Un dispositif d'inhalation selon la revendication 4 et l'une quelconque des revendications 9 à 14, dans lequel la course du piston (12) dans le cylindre (10) définissant la chambre principale entre ses positions avancée et reculée est réglable.

16. Un dispositif d'inhalation selon l'une quelconque des revendications 9 à 15, dans lequel la chambre d'entrée a une partie formant paroi cylindrique, le réservoir étant relié à la chambre d'entrée par un passage qui est orienté sensiblement périphériquement par rapport à la partie formant paroi cylindrique.

17. Un dispositif d'inhalation selon l'une quelconque des revendications 8 à 16, dans lequel l'embout buccal fait partie intégrante d'une unité (23) comprenant le passage d'expiration (25) et le clapet de retenue (27) disposé dans celui-ci.

18. Un dispositif d'inhalation selon l'une quelconque des revendications 5 à 17, comportant en outre un dispositif à levier pour faire passer le piston, à l'encontre de la force de sollicitation du ressort, de sa position avancée à sa position reculée.
